# FASCICULE DE BREVET EUROPEEN

(11) **EP 2 521 500 B1**
(45) Date de publication et mention de la délivrance du brevet: **19.03.2014**
(21) Numéro de dépôt: 11703720.0
(22) Date de dépôt: 05.01.2011
(51) Int. Cl.: A61B 17/70

(54) **DISPOSITIF DE FIXATION VERTEBRALE**
VERTEBRALE BEFESTIGUNGSVORRICHTUNG
VERTEBRAL ATTACHMENT DEVICE

(30) Priorité: 06.01.2010 FR 1000040
(43) Date de publication de la demande: 14.11.2012
(73) Titulaire: Implanet, Société Anonyme, 33650 Martillac (FR)
(72) Inventeur: BACCELI, Christian, F-33650 Saucats (FR); LE COUEDIC, Régis, F-33800 Bordeaux (FR)
(74) Mandataire: Benech, Frédéric
(86) Numéro de dépôt international: PCT/FR2011/000005
(87) Numéro de publication internationale: WO 2011/083261

(56) Documents cités:
- EP-A1- 2 047 813
- WO-A1-2009/013397
- FR-A1- 2 842 724
- FR-A1- 2 890 850
- US-B1- 6 554 831

## Description

La présente invention concerne un dispositif de fixation vertébrale pour maintien d'une vertèbre rachidienne sur une tige, comportant un corps de fixation sur la tige, une bande souple de liaison de ladite vertèbre avec le corps de fixation et des moyens de blocage réglable de la bande souple sur le corps de fixation.

Elle trouve une application particulièrement importante bien que non exclusive dans le domaine du redressement de la colonne vertébrale d'un patient présentant une courbure anormale.

Dans ce cas, les vertèbres n'étant pas alignées correctement les unes par rapport aux autres par rapport à l'axe vertébral, elles présentent des inclinaisons entre elles.

A certains endroits, les bords latéraux des vertèbres vont donc être d'un côté rapprochés les uns des autres, et de l'autre côté éloignés les uns des autres.

Afin de redresser l'ensemble, il est connu de remettre à une distance sensiblement équivalente les bords latéraux des vertèbres de part et d'autre de la colonne vertébrale, par le biais de tiges reliant entre elles, soit des vis, que l'on insère dans les vertèbres elles-mêmes, soit des crochets, que l'on introduit le long du canal rachidien.

De tels dispositifs présentent cependant des inconvénients.

L'utilisation de vis n'est tout d'abord possible que si les vertèbres sont en bon état et/ou suffisamment larges au niveau de la fixation.

L'utilisation de crochets est quant à elle très délicate puisque l'opérateur ne doit pas toucher la moelle épinière sous peine de paralysie du patient.

Pour pallier à ces inconvénients il a été proposé (FR 02 09317 publié sous le N° 2 842 724) un système permettant d'éviter les vis de fixation ou les crochets.

Le système comprend un lien flexible de fixation de la vertèbre sur une pièce de liaison elle-même fixée à la tige de redressement.

Des moyens de blocage du lien flexible par refermeture de la pièce de liaison sur la tige sont prévus. Ce système présente cependant ici encore des inconvénients.

Il nécessite en effet, d'une part une articulation de la pièce de liaison de façon à permettre l'insertion latérale de la tige et du lien souple, et d'autre part une fixation du lien sur la pièce pour permettre un tel blocage après refermeture de la pièce sur la tige.

On connaît également (EP 2 047 813) un dispositif de fixation vertébrale comportant un corps de fixation d'une bande souple sur une tige, en forme de mâchoire articulée.

Chaque branche de la mâchoire est munie d'un orifice de passage d'un des deux brins d'une boucle de la bande, propres à être bloqués en fond de ladite mâchoire par la tige une fois la mâchoire refermée et vissée. Le dispositif articulé est fragile et ne permet pas un réglage facile de la bande souple.

Le même document décrit un autre système, formée cette fois-ci d'une pièce cylindrique munie d'un orifice inférieur de passage de la boucle, et de deux fentes supérieures de sortie des brins libres permettant le coinçage des deux brins vers la face interne du cylindre par le biais d'un élément demi cylindrique, de hauteur sensiblement égale à la longueur de la pièce, et d'une vis de compression dudit élément sur les brins de la boucle.

Un tel système qui ne concerne visiblement pas le maintien d'une vertèbre sur une tige cylindrique, ne permet notamment pas une mise en place facile, nécessitant notamment un outil de guidage.

On connaît aussi (WO 2009/013397) un dispositif de fixation vertébrale pour système de correction des courbures anormales du rachis utilisant un collier de serrage constitué par une bande souple crantée passant dans une pièce pliée en deux de fixation entre ses deux branches d'une tige avec lumière d'accrochage du collier par crantage. Un tel dispositif est difficile à régler, et une fois serré ne permet pas une reprise dudit serrage.

La présente invention vise à fournir un dispositif de fixation vertébrale répondant mieux que ceux antérieurement connus aux exigences de la pratique, notamment en ce qu'elle va permettre une plus grande flexibilité, une meilleure solidité du fait d'une absence d'articulation mécanique toujours susceptible de blocage, et en ce qu'elle présente des possibilités de réglage très améliorées, et ce pour un coût moindre.

Dans ce but, l'invention propose essentiellement un dispositif de fixation vertébrale pour maintien d'une vertèbre rachidienne sur une tige cylindrique, comportant un corps de fixation sur la tige, une bande souple de liaison de ladite vertèbre avec le corps de fixation et des moyens de blocage réglable de la bande souple sur le corps de fixation, le corps de fixation étant de section transversale en forme de U, de passage de la tige entre la paroi de fond du U et les moyens de blocage réglable, lesdits moyens de blocage réglable étant formés par une pièce de liaison reliant les extrémités en vis à vis des deux branches du U, les dites branches comprenant chacune un évidement en vis à vis situé du coté du fond du U, à savoir un premier évidement pour le passage des extrémités libres et un deuxième évidement de l'autre côté pour le passage de la bande propre à former une boucle de fixation sur la vertèbre,
caractérisé en ce que le corps est formé d'une seule pièce, et
en ce que la pièce de liaison est formée par une vis de serrage munie d'un côté d'une tête de passage dans une première branche du U, ladite tête comportant une partie tronconique agencée pour comprimer la tige au fur et à mesure de son vissage, et comprimer ainsi les deux extrémités de la bande souple contre la paroi, et de l'autre côté d'une extrémité de vissage sur la branche du U opposée.

Par corps d'une seule pièce, on entend un monobloc rigide.

Lors du serrage pour fixation, le fait que la boucle présente deux brins libres initialement non comprimés la tige étant en position, à savoir deux brins l'un contre l'autre non comprimés en vis à vis de la tige, autorise un glissement relatif du lien par rapport au corps de fixation, et de ce fait un ajustage d'une grande souplesse non générateur de contraintes en cisaillement sur la vertèbre et/ou sur le corps de fixation. Celui-ci peut ainsi également rester positionné face au chirurgien et à l'outil de serrage de la vis.

Une telle possibilité de glissement adaptatif, non autorisé par l'art antérieur soit du fait de la fixation du lien sur le corps de fixation, soit du fait d'une articulation dont les mâchoires, pour être fixées l'une à l'autre, entraînent nécessairement une compression initiale sur la tige même, apparaissait comme un facteur d'imprécision et de mauvaise tenue du lien sur la tige.

Avec l'invention et grâce notamment à l'effet de coin obtenu par la pièce de liaison, il n'en est rien.

Dans des modes de réalisation avantageux, on a de plus recours à l'une et/ou à l'autre des dispositions suivantes :
- la paroi de fond du U est en forme de demi cylindre de forme complémentaire à celle de la tige, terminé de part et d'autre par des rebords longitudinaux autorisant un clipsage de la tige dans le fond du U grâce à une déformation des branches.

Il est dès lors possible pour le chirurgien de préfixer la tige avec le corps précisément, tout en autorisant encore des possibilités de glissements longitudinaux du corps le long de la tige. Le clipsage est en effet léger et n'a qu'un rôle de maintien, sans exercer de pression sur la barre et sur les brins en vis à vis dans le fond du U ;
- la tête de la vis de serrage comporte de plus un épaulement propre à coopérer avec la face externe de la première branche du U.

Un tel épaulement, quand il arrive en butée, indique ainsi au chirurgien qu'il a obtenu le serrage adéquat. Il peut de plus avoir un rôle de serrage complémentaire.

Les branches du U étant libres la pièce possède en effet lors du serrage, une certaine flexibilité, la distance entre les extrémités des branches du U étant dès lors légèrement modulable, avec effet de pince ;
- le corps de fixation, la tige et la pièce de liaison sont en titane ;
- le corps de fixation est en matériau polymère :
- le fond du U comporte des crans de blocage complémentaire par frottement ;
- les crans sont parallèles à l'axe longitudinal du fond du U (ou au plan longitudinal de symétrie de la pièce), à sommet plat ;
- les crans sont des crans anti-retour, parallèles en plan longitudinal du U, et présentant des arêtes formant des angles opposés ou perpendiculaires au sens de glissement vers le resserrement de la boucle ;
- la bande souple est une tresse en polymère.

L'invention concerne également un système de redressement d'une colonne vertébrale comprenant au moins deux dispositifs tels que décrits ci-dessus et au moins une tige.

Avantageusement il comprend deux tiges et au moins quatre dispositifs.

La demande expose également une méthode de fixation d'une vertèbre rachidienne sur une tige mettant en oeuvre un dispositif et/ou système tels que décrits ci-dessus. Cette méthode n'est pas revendiquée.

La demande expose également une méthode, non revendiquée, de fixation d'une vertèbre rachidienne sur une tige, dans laquelle
- on passe une bande souple autour d'une partie de la vertèbre pour former une boucle enserrant ladite partie dont les extrémités libres sortent du coté retenu pour fixation sur la tige, et
- on passe lesdites extrémités libres dans deux évidements d'un corps d'une seule pièce de fixation sur la tige, de section transversale en forme de U, chaque évidemment étant situé dans une branche respective du U et du coté du fond du U, caractérisé en ce que
- on introduit la tige par clipsage via l'ouverture du U, de sorte que les extrémités libres sont prises entre la tige et le fond du U,
- on ajuste la tension de la boucle sur la partie de vertèbre en tirant simultanément sur les brins, et
- on vient progressivement comprimer la tige contre les brins pour les bloquer en translation par des moyens de blocage réglable, lesdits moyens de blocage réglable étant formés par une pièce de liaison reliant les extrémités en vis à vis des deux branches du U.

Avantageusement la pièce de liaison étant formée par une vis de serrage munie d'un côté d'une tête de passage dans une première branche du U, ladite tête comportant une partie tronconique dirigée vers l'autre branche et de l'autre côté d'une extrémité de vissage sur la branche du U opposée, on visse ladite vis de serrage de sorte qu'on comprime la tige par la partie tronconique au fur et à mesure de son vissage, et on comprime ainsi les deux extrémités de la bande souple contre la paroi du fond du U.

Egalement avantageusement on arrête le vissage par blocage d'un épaulement de la tête de vis sur la face externe de la première branche du U.

Dans un mode de réalisation avantageux, on forme au moins deux boucles en passant leurs extrémités respectives dans au moins deux corps de fixation tels que décrits ci-avant puis on les clipse et on les met en compression par vissage sur une même tige.

L'invention sera mieux comprise à la lecture de la description qui suit de modes de réalisation donnés ci-après à titre d'exemples non limitatifs.

La description se réfère aux dessins qui l'accompagnent dans lesquels :
- La figure 1 est une vue en perspective du dispositif selon l'invention.
- La figure 2 montre un mode de réalisation du corps, des moyens de blocage et de la tige d'union désolidarisés les uns des autres, selon l'invention.
- La figure 3 est une vue en coupe selon III III du dispositif de la figure 1.
- La figure 4 montre, en vue de dessus, le dispositif de la figure 1 fixé sur une vertèbre.

Dans la suite de la description on utilisera les mêmes numéros de références pour désigner les mêmes éléments.

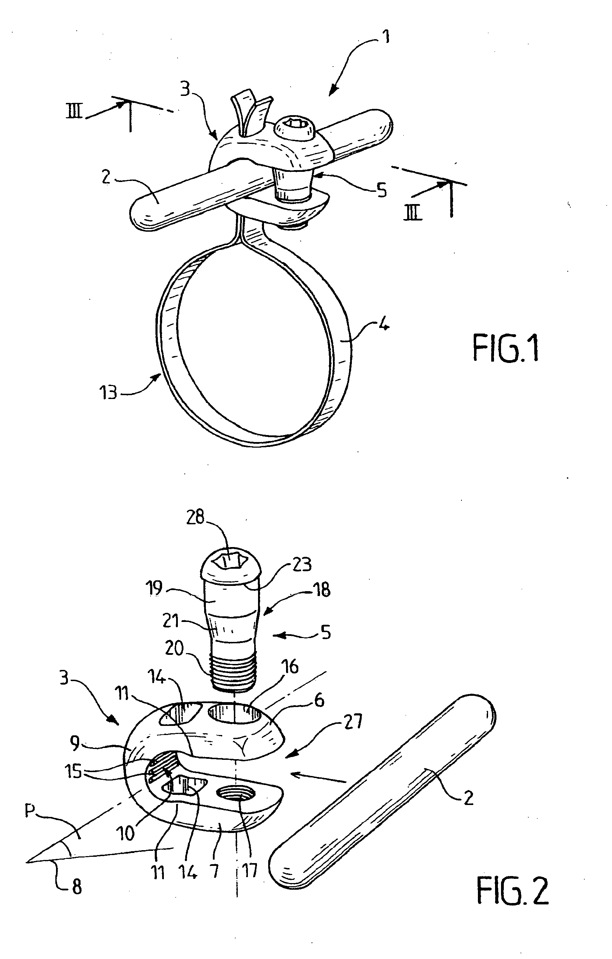

La figure 1 montre un dispositif 1 de fixation vertébrale pour maintien d'une vertèbre rachidienne (non représentée) sur une tige cylindrique 2.

Le dispositif comporte un corps 3 de fixation sur la tige et une bande souple 4 en polymère tressé, par exemple en polyester de 6mm large et de trente centimètres de long.

Il comprend également des moyens 5 de blocage réglable de la bande souple sur le corps de fixation 3.

Plus précisément en référence à la figure 2, le corps 3 est formé par une pièce d'un seul tenant formant une pince présentant une section transversale en forme de U, ledit U comprenant deux branches épaisses 6 et 7 de section transversale sensiblement en forme de demi ovale, symétriques par rapport à un plan longitudinal 8, et reliées entre elles par une partie de liaison 9 en forme de demi anneau torique formant d'un côté le fond en demi cylindre 10 du U, et de l'autre côté une paroi externe arrondie de surface torique.

La paroi de fond du U est de forme complémentaire à celle de la tige 2 ou sensiblement complémentaire, et comprend des lèvres ou rebords longitudinaux 11 autorisant un clipsage de la tige dans le fond du U une fois la tresse 4 passée en double épaisseur pour former la boucle 13 (voir figure 1).

Chaque branche 6, 7 comporte un évidement 14 , par exemple en forme de fente large, par exemple 5 à 10 fois plus large que l'épaisseur de la tresse pour faciliter son introduction lors de l'opération.

Le fond 10 du U comporte de plus des crans 15 de blocage, anti-retour, parallèles au plan longitudinal 8 et présentant de façon connue en elle-même des arêtes formant des angles opposés ou perpendiculaires au sens de glissement, s'opposant au desserrement de la boucle une fois que le serrement est exercé.

Chaque branche 6 et 7 comporte un orifice cylindrique respectivement 16 et 17 de passage des moyens 5 de blocage, à savoir un alésage 16 de diamètre D et un orifice cylindrique taraudé 17 de vissage de diamètre d < D.

Les moyens 5 de blocage sont formés par une pièce de liaison 18, ou vis, munie d'un côté d'une tête 19 de passage dans l'alésage 16 du U, et de l'autre côté d'une extrémité 20 de vissage dans l'orifice taraudé 17.

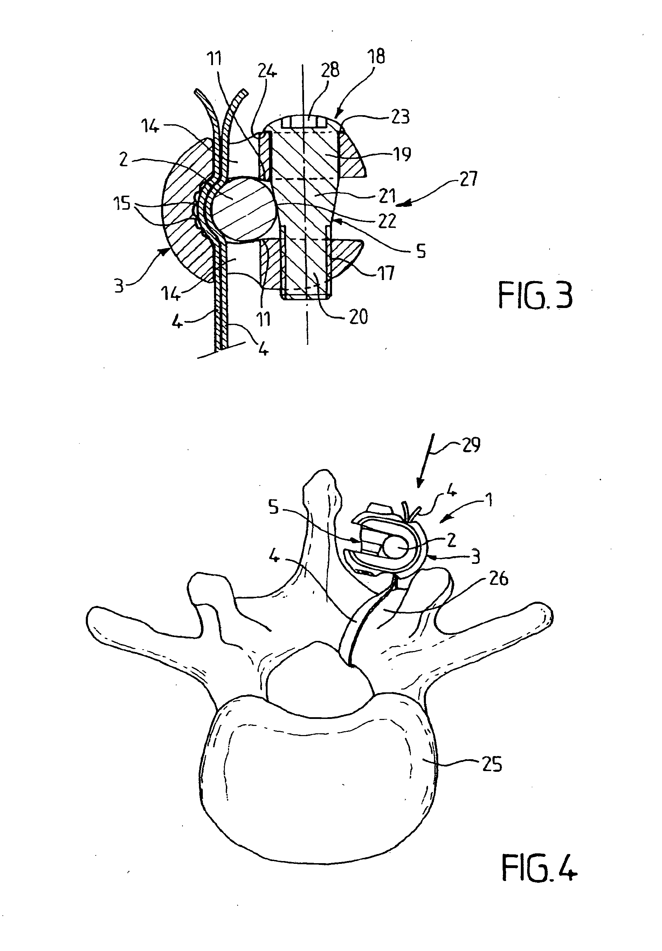

La tête 19 de la pièce 18 comprend une partie supérieure cylindrique qui coopère à frottement doux avec l'alésage 16, ladite partie supérieure étant connectée de façon solidaire avec une partie inférieure tronconique 21, se rétrécissant vers le bas et agencée pour comprimer (appui 22 sur la figure 3) la tige 2 au fur et à mesure du vissage de la pièce.

Dans le mode de réalisation plus particulièrement décrit ici, la tête de la vis comporte de plus un épaulement 23, par exemple tronconique, propre à coopérer avec la face externe 24 de la première branche du U et à servir de butée d'arrêt du vissage.

On va maintenant décrire en référence aux figures 2, 3 et 4 la mise en place du dispositif 1 sur la vertèbre 25 (cf. figure 4).

Le dispositif 1 décrit ci-avant permet de connecter mécaniquement la tresse souple 4 sur la tige d'union métallique 2 avec la vertèbre 25.

Cet implant est particulièrement indiqué dans le cadre d'une chirurgie du rachis de type scoliose.

La tresse 4 est préalablement introduite dans le corps 3 tout en venant insérer en boucle une partie 26 de l'anatomie rachidienne (ici la lame transverse, arc postérieur).

La même opération est réalisée sur la ou les vertèbres d'à côté que l'on souhaite repositionner les unes par rapport aux autres, pour ici réparer la scoliose.

Une fois ces boucles réalisées, la tige d'union 2 est introduite par clipsage par le côté via les ouvertures 27 des U des corps 3.

On va décrire uniquement ci-après la fixation d'un seul corps, les fixations des autres étant effectuées de la même façon une fois leur position respective fixée le long de la tige.

La vis 18 est insérée dans l'alésage 16 puis commence à être vissée dans l'alésage 17.

Le vissage est effectué par le chirurgien qui a accès aux têtes de vis chacune munie d'un orifice et/ou évidement de vissage 28 connu en lui même (voir flèche 29 sur la figure 4).

Une fois l'ensemble prépositionné et alors qu'il reste du jeu sur les tresses, celles-ci sont tirées de façon à venir serrer les boucles sur les lames des vertèbres respectives.

Les arêtes anti-retour 15 permettent le serrage dans un sens et empêchent le desserrage dans l'autre lorsque les deux extrémités de la bande souple restent encore un peu mobile.

Puis le vissage des vis 19 permet aux parties coniques 21 de venir progressivement en appui sur la tige d'union 2 jusqu'à verrouiller l'ensemble en bloquant pour chaque dispositif par serrage la tresse 4 entre le fond du U et la tige d'union.

Comme il va de soi et comme il résulte également de ce qui précède, la présente invention n'est pas limitée aux modes de réalisation plus particulièrement décrits. Elle en embrasse au contraire toutes les variantes et notamment celles où deux ou plusieurs tiges sont fixées à la suite ou de part et d'autre de la colonne vertébrale.

## Revendications

1. Dispositif (1) de fixation vertébrale pour maintien d'une vertèbre rachidienne (25) sur une tige cylindrique(2), comportant un corps (3) de fixation sur la tige, une bande souple (4) de liaison de ladite vertèbre avec le corps de fixation et des moyens (5) de blocage réglable de la bande souple sur le corps de fixation, le corps (3) de fixation étant de section transversale en forme de U, de passage de la tige entre la paroi (10) de fond du U et les moyens de blocage réglable, lesdits moyens (5) de blocage réglable étant formés par une pièce de liaison (16) reliant les extrémités en vis à vis des deux branches (6, 7) du U, les dites branches (6, 7) comprenant chacune un évidement (14) en vis à vis situé du coté du fond du U, à savoir un premier évidement pour le passage des extrémités libres et un deuxième évidement de l'autre côté pour le passage de la bande propre à former une boucle de fixation sur la vertèbre,
**caractérisé en ce que** le corps est formé d'une seule pièce, et
**en ce que** la pièce de liaison (18) est formée par une vis de serrage munie d'un côté d'une tête (19) de passage dans une première branche (6) du U, ladite tête comportant une partie tronconique (21) agencée pour comprimer la tige (2) au fur et à mesure de son vissage, et comprimer ainsi les deux extrémités de la bande souple contre la paroi, met de l'autre côté d'une extrémité (20) de vissage sur la branche du U opposée.

2. Dispositif selon la revendication 1, **caractérisé en ce que** la paroi (10) de fond du U est en forme de demi cylindre de forme complémentaire à celle de la tige, terminé de part et d'autre par des rebords longitudinaux (11) autorisant un clipsage de la tige dans le fond du U grâce à une déformation des branches.

3. Dispositif selon l'une quelconque des revendications précédentes, **caractérisé en ce que** la tête de la vis de serrage comporte de plus un épaulement (23) propre à coopérer avec la face externe 24 de la première branche du U.

4. Dispositif selon l'une quelconque des revendications précédentes, **caractérisé en ce que** le corps (3) de fixation, la tige (2) et la pièce de liaison (18) sont en titane.

5. Dispositif selon l'une quelconque des revendications 1 à 4, **caractérisé en ce que** le corps (3) de fixation est en matériau polymère.

6. Dispositif selon l'une quelconque des revendications précédentes, **caractérisé en ce que** le fond (10) du U comporte des crans (15) de blocage complémentaire par frottement.

7. Dispositif selon la revendication 6, **caractérisé en ce que** les crans (15) sont parallèles à l'axe longitudinal du fond du U, à sommet plat.

8. Dispositif selon la revendication 7, **caractérisé en ce que** les crans sont des crans anti-retour, parallèles à l'axe longitudinal du fond du U, et présentant des arêtes formant des angles opposés ou perpendiculaires au sens de glissement vers le resserrement de la boucle.

9. Dispositif selon l'une quelconque des revendications précédentes, **caractérisé en ce que** la bande souple (4) est une tresse en polymère.

10. Système de redressement d'une colonne vertébrale sur une tige comprenant au moins deux dispositifs (1) selon l'une quelconque des revendications précédentes, **caractérisé en ce qu'**il comporte ladite tige (2) cylindrique sur laquelle sont fixés lesdits dispositifs.

11. Système selon la revendication 10, **caractérisé en ce qu'**il comporte deux tiges (2) et au moins quatre dispositifs (1).

## Patentansprüche

1. Wirbelsäulenfixierungsvorrichtung (1) zum Halten eines Wirbelsäulenwirbels (25) an einem zylindrischen Stift (2), umfassend einen Körper (3) für die Fixierung an dem Stift, ein flexibles Band (4) für die Verbindung des Wirbelknochens mit dem Fixierungskörper und Mittel (5) für die einstellbare Arretierung des flexiblen Bandes an dem Fixierungskörper, wobei der Fixierungskörper (3) einen U-förmigen Querschnitt für die Durchführung des Stifts zwischen der Bodenwand (10) des U und den Mitteln für die einstellbare Arretierung aufweist, wobei die Mittel (5) für die einstellbare Arretierung durch ein Verbindungsstück (18) gebildet sind, das die gegenüberliegenden Enden der zwei U-Schenkel (6, 7) verbindet, wobei die Schenkel (6, 7) jeweils eine entgegengesetzte Ausnehmung (14) aufweisen, welche auf der Seite des U-Bodens angeordnet ist, und zwar eine erste Ausnehmung für die Durchführung der freien Enden und eine zweite Ausnehmung auf der anderen Seite für die Durchführung des Bands, das geeignet ist, eine Schleife zur Fixierung an dem Wirbelknochen zu bilden,
**dadurch gekennzeichnet,**
**dass**
- der Körper einstückig ausgebildet ist und
- das Verbindungsstück (18) durch eine Klemmschraube gebildet ist, die auf der einen Seite mit einem durch einen ersten Schenkel (6) des U durchgeführten Kopf (19) versehen ist, wobei der Kopf einen kegelstumpfförmigen Abschnitt (21) aufweist, der angeordnet ist, um den Stift (2) bei fortschreitender Verschraubung zusammenzudrücken und somit die beiden Enden des flexiblen Bands gegen die Wand zusammenzudrücken, und auf der anderen Seite ein Ende (20) für die Verschraubung an dem entgegengesetzten U-Schenkel aufweist.

2. Vorrichtung nach Anspruch 1,
**dadurch gekennzeichnet,**
**dass** die Bodenwand (10) des U die Form eines komplementär zu der Form des Stiftes ausgebildeten Halbzylinders aufweist, der beidseitig mit längsseitigen Rändern (11) endet, welche durch Verformung der Schenkel ein Einclipsen des Stifts in den U-Boden ermöglichen.

3. Vorrichtung nach einem beliebigen der vorhergehenden Ansprüche,
**dadurch gekennzeichnet,**
**dass** der Kopf der Klemmschraube ferner einen Absatz (23) aufweist, der geeignet ist, mit der Außenfläche (24) des ersten U-Schenkels zusammenzuwirken.

4. Vorrichtung nach einem beliebigen der vorhergehenden Ansprüche,
**dadurch gekennzeichnet,**
**dass** der Fixierungskörper (3), der Stift (2) und das Verbindungsstück (18) aus Titan bestehen.

5. Vorrichtung nach einem beliebigen der Ansprüche 1 bis 4,
**dadurch gekennzeichnet,**
**dass** der Fixierungskörper (3) aus einem Polymermaterial besteht.

6. Vorrichtung nach einem beliebigen der vorhergehenden Ansprüche,
**dadurch gekennzeichnet,**
**dass** der U-Boden (10) Rasten (15) für eine ergänzende Arretierung durch Reibung aufweist.

7. Vorrichtung nach Anspruch 6,
**dadurch gekennzeichnet,**
**dass** die Rasten (15) mit flachem Scheitel parallel zu der Längsachse des U-Bodens verlaufen.

8. Vorrichtung nach Anspruch 7,
**dadurch gekennzeichnet,**
**dass** es sich bei den Rasten um Sperrrasten handelt, die parallel zu der Längsachse des U-Bodens verlaufen und Kanten aufweisen, welche Winkel bilden, die entgegengesetzt oder senkrecht zur Richtung des Gleitens zur Schleifenverengung hin sind.

9. Vorrichtung nach einem beliebigen der vorhergehenden Ansprüche,
**dadurch gekennzeichnet,**
**dass** es sich bei dem flexiblen Band (4) um ein Polymergeflecht handelt.

10. System zum Richten einer Wirbelsäule an einem Stift mit mindestens zwei Vorrichtungen (1) nach einem beliebigen der vorhergehenden Ansprüche,
**dadurch gekennzeichnet,**
**dass** es den zylindrischen Stift (2) aufweist, an dem die Vorrichtungen fixiert sind.

11. System nach Anspruch 10,
**dadurch gekennzeichnet,**
**dass** es zwei Stifte (2) und mindestens vier Vorrichtungen (1) aufweist.

## Claims

1. Vertebral fastening device (1) for holding a rachidian vertebra (25) on a cylindrical rod (2), having a fastening body (3) on the rod, a flexible band (4) for linking said vertebra with the fastening body and means (5) for adjustable locking of the flexible band on the fastening body, the fastening body (3) being of U-shaped transverse section, for passage of the rod between the wall (10) of the bottom of the U and the adjustable locking means, said adjustable locking means (5) being formed by a linking part (18) connecting the facing ends of the two limbs (6, 7) of the U, said limbs (6, 7) each comprising a facing recess (14) situated on the side of the bottom of the U, namely a first recess for passage of the free ends and a second recess on the other side for passage of the band capable of forming a fastening loop on the vertebra,
**characterised in that** the body is formed of a single part, and
**in that** the linking part (18) is formed by a clamping screw provided on one side with a head (19) for passage through a first limb (6) of the U, said head having a part (21) in the shape of a truncated cone arranged to compress the rod (2) as it is screwed up, and thus compress the two ends of the flexible band against the wall, and on the other side with an end (20) for screwing to the opposite limb of the U.

2. Device according to claim 1, **characterised in that** the wall (10) of the bottom of the U is in the shape of a half cylinder complementing that of the rod, terminating on either side in longitudinal raised edges (11) allowing the rod to be clipped into the bottom of the U by virtue of a deformation of the limbs.

3. Device according to any one of the preceding claims, **characterised in that** the head of the clamping screw also has a shoulder (23) capable of cooperating with the external face (24) of the first limb of the U.

4. Device according to any one of the preceding claims, **characterised in that** the fastening body (3), the rod (2) and the linking part (18) are made of titanium.

5. Device according to any one of claims 1 to 4, **characterised in that** the fastening body (3) is made of polymer material.

6. Device according to any one of the preceding claims, **characterised in that** the bottom (10) of the U has notches (15) for complementary locking through friction.

7. Device according to claim 6, **characterised in that** the notches (15) are parallel to the longitudinal axis of the bottom of the U, with a flat top.

8. Device according to claim 7, **characterised in that** the notches are anti-return notches, parallel to the longitudinal axis of the bottom of the U, and exhibiting ridges forming angles opposing or perpendicular to the direction of sliding in the tightening direction of the loop.

9. Device according to any one of the preceding claims, **characterised in that** the flexible band (4) is a braided polymer.

10. System for straightening a vertebral column on a rod comprising at least two devices (1) according to any one of the preceding claims, **characterised in that** it comprises said cylindrical rod (2) on which said devices are fastened.

11. System according to claim 10, **characterised in that** it comprises two rods (2) and at least four devices (1).
